# EUROPEAN PATENT APPLICATION

(11) **EP 1 064 908 A1**
(43) Date of publication of application: **03.01.2001**
(21) Application number: 00201176.5
(22) Date of filing: 31.03.2000
(51) Int. Cl.: A61K 7/00

(54) **Solid cosmetic product in water-in-oil non-ionic emulsion**

(30) Priority: 02.07.1999 IT MI991464
(71) Applicant: INTERCOS ITALIA S.p.A., 20122 Milano (IT)
(72) Inventor: Avalle, Nadia, 20124 Milano (IT)
(74) Representative: Mittler, Enrico

(57) **Abstract**

Solid cosmetic product in water-in-oil non-ionic emulsion, comprising:
e) from 0.5 to 20% of a non-ionic emulsifier or of a mixture of non-ionic emulsifiers of the carboxyl-alkyl-polyglycerols family :

   R-O-CH₂-CH(-OR)-CH₂-O-(-CH₂-CH(-OR)-CH₂-O-R)ₙ

   where n= 1 to 50, R= H and/or C₁₈ to C₃₃;
f) from 5 to 90% of a lipid phase with dropping point higher than 60°C;
g) from 1 to 80% of a water phase;
from 1 to 80% of a powder phase.

## Description

The present invention concerns a solid cosmetic product in water-in-oil (W/O) non-ionic emulsion.

In the field of cosmetics emulsions represent a typology of presentation of the cosmetic product that allows to carry out both a protective as well as functional action in the topic treatment of the epidermis.

The realisation of an emulsion is fundamentally determined by the emulsifying system being used, that can avail of non-ionic, cationic, anionic and amphoteric type molecules.

Each emulsifier determines the optimal affinity between phases, delicacy and compatibility with the epidermis.

Specific object of the present invention is to obtain a solid cosmetic product in water-in-oil (W/O) emulsion with a high cosmetic delicacy, utilisable both for makeup as well as for the treatment of the skin.

According with the invention such object has been attained with a cosmetic product comprising:
a) from 0.5 to 20% of an non-ionic emulsifier or of a mixture of non-ionic emulsifiers of the carboxyl-alkyl-polyglycerols family:

   R-O-CH₂-CH(-OR)-CH₂-O-(-CH₂-CH(-OR)-CH₂-O-R)ₙ

   where n= 1 to 50, R= H and/or C₁₈ to C₃₃;
b) from 5 to 90% of a lipid phase with dropping point higher than 60°C;
c) from 1 to 80% of a water phase;
d) from 1 to 80% of a powder phase.

Qualifying is the use of the polyglycerols for their high efficiency in the maintenance of the micellar structure through time and during the manufacture process and for their high dermal compatibility.

The lipid phase can be made of natural and/ or synthetic liquid and waxy esters, liquid and waxy alcohols, silicone oils and waxes, polyalfaolefines with variable molecular weight, polyethylene waxes, microcrystalline waxes, and more.

The water phase can in turn consist of different wetting agents, as glycerine, butylenic glycol, hexylene glycol, from PEG 4 to PEG 100, sorbitol, manitol, saccharose, fructose, different electrolytes as NaCl, MgSO4, Na-lactate, KCl, and other more.

The powder phase can be semi-transparent or coloured with different covering power, made of different excipients powders, as talc, kaolin, mica, rice starch, modified starches, reticulated polymethyl-metacrylate, polyamides, tricalcic phosphate, silica, lauroyl lysine, barium sulphate, boron nitride, and of colour pigment of different nature as titanium dioxide, iron oxides, ultramarine blue, zinc oxide, D&C red 30, D&C red 7 Al Lake, D&C yellow 5 Al Lake.

Only as an example and without any limiting intention some formulations considered to be as suitable for specific cosmetic products are hereafter reported.

| Example n. 1: Compact foundation cream | |
|---|---|
| Ingredients | % |
| Trioctanoate trimethylpropane | 5.00 |
| Iso hexadecane | 6.50 |
| Squalene | 2.00 |
| Hydrogenated vegetable oil | 3.50 |
| Distearate polyglyceryl | 6.00 |
| Vergin wax | 2.00 |
| Candle wax | 3.30 |
| Hydrogenated castor oil | 2.00 |
| Pentacyclomethycon | 4.60 |
| Caprylic/capric-glycerids | 6.10 |
| Micro-crystalline wax | 2.00 |
| Soy sterol | 0.50 |
| Water | 15.20 |
| Glycerine | 5.00 |
| Butylen glycol | 4.80 |
| MgSO4 | 0.30 |
| Na-lactate | 0.80 |
| Hydroxyl-ethyl-cellulose | 0.20 |
| Potassium sorbate | 0.20 |
| Xantam rubber | 0.20 |
| Titanium dioxide | 8.50 |
| Iron oxides | 4.20 |
| Ultramarine Blue | 0.30 |
| Zinc Oxide | 2.00 |
| Nylon-12 | 4.00 |
| Polymethylmetacrylate | 3.00 |
| Rice starch | 3.80 |
| Silica | 2.00 |
| Mica | 2.00 |
| Total | 100.00 |

| Example n. 2: Compact protective cream | |
|---|---|
| Ingredients | % |
| Cetyl Octanoate | 15.60 |
| Hydrogenated vegetable oil | 10.10 |
| Rice wax | 2.00 |
| Cholesteryl-hydroxyl-stearate | 0.50 |
| Hydrogenated castor oil | 3.20 |
| Soy sterol | 0.30 |
| Pentacyclomethycon | 16.59 |
| Distearate polyglyceryl | 8.00 |
| Caprylic/capric glycerids | 6.20 |
| Micro-crystalline wax | 2.50 |
| Water | 21.00 |
| Glycerine | 6.00 |
| Sorbitol | 3.50 |
| Mannitol | 2.00 |
| Xantam rubber | 0.20 |
| Potassium sorbate | 0.30 |
| MgSO4 | 0.40 |
| Na-lactate | 0.60 |
| Na jaluronate | 0.01 |
| Alfa-Bisabol | 0.20 |
| Matricaria extract | 0.80 |
| Total | 100.00 |

| Example n. 3: Stick foundation cream | |
|---|---|
| Ingredients | % |
| Cetyl iso nonanoate | 4.85 |
| Cetyl octanoate | 10.50 |
| Squalene | 3.70 |
| Pentacyclomethycon | 4.10 |
| Iso hexadodecanoate | 3.90 |
| Hydrogenated vegetable oil | 2.05 |
| Rice wax | 3.00 |
| Candle wax | 2.80 |
| Distearate polyglyceryl | 6.00 |
| Soy sterol | 0.30 |
| Caprylic/capric glycerids | 4.00 |
| Ceresine | 2.10 |
| Hydrogenated castor oil | 2.00 |
| Micro-crystalline wax | 3.60 |
| Water | 14.10 |
| Glycerine | 6.00 |
| Butylen glycol | 2.00 |
| MgSO4 | 0.40 |
| Sorbitol | 1.00 |
| Potassium sorbate | 0.20 |
| Hydroxyl-ethyl-cellulosa | 0.20 |
| Xantam rubber | 0.20 |
| Titanium dioxide | 8.50 |
| Iron oxides | 3.00 |
| Ultramarine blue | 0.50 |
| Zinc oxide | 1.00 |
| Borum nitride | 1.00 |
| Lauroyl lysine | 1.50 |
| Rice starch | 3.00 |
| Silica | 2.50 |
| Talc | 2.00 |
| Total | 100.00 |

## Claims

1. Solid cosmetic product in water-in-oil non-ionic emulsion, comprising:
a) from 0.5 to 20% of a non-ionic emulsifier or of a mixture of non-ionic emulsifiers of the carboxyl-alkyl-polyglycerols family:
R-O-CH₂-CH(-OR)-CH₂-O-(-CH₂-CH(-OR)-CH₂-O-R)ₙ
where n= 1 to 50, R= H and/or C₁₈ to C₃₃;
b) from 5 to 90% of a lipid phase with dropping point higher than 60°C;
c) from 1 to 80% of a water phase;
d) from 1 to 80% of a powder phase.

2. Solid cosmetic product according to claim 1, in which the lipid phase comprises components selected among liquid and waxy esters, natural and/ or synthetic, liquid and waxy alcohols, silicone oils and waxes, polyalfaolefines with variable molecular weight, polyethylene waxes, microcrystalline waxes.

3. Solid cosmetic product according to claim 1, in which the water phase comprises wetting agents and electrolytes.

4. Solid cosmetic product according to claim 3, in which the wetting agents are selected among glycerine, butylenic glycol, hexylene glycol, from PEG 4 to PEG 100, sorbitol, manitol, saccharose, fructose.

5. Solid cosmetic product according to claim 3, in which the electrolytes are selected among NaCl, MgSO4, Na-lactate, KCl.

6. Solid cosmetic product according to claim 1, in which the powder phase comprises excipient and pigment powder.

7. Solid cosmetic product according to claim 6, in which the excipient powders are selected among talc, kaolin, mica, rice starch, modified starches, reticulated polymethylmetacrylate, polyamides, tricalcic phosphate, silica, lauroyl lysine, barium sulphate, boron nitride.

8. Solid cosmetic product according to claim 6, in which the pigment are selected among titanium dioxide, iron oxides, ultramarine blue, zinc oxide, D&C red 30, D&C red 7 Al Lake, D&C yellow 5 Al Lake.
